# EUROPEAN PATENT APPLICATION

(11) **EP 3 861 997 A1**
(43) Date of publication of application: **11.08.2021**
(21) Application number: 21155066.0
(22) Date of filing: 03.02.2021
(51) Int. Cl.: A61K 31/40, A61K 45/06, A61P 25/00, A61P 25/28, A61P 35/00

(54) **COMPOSITION FOR INCREASING PERMEABILITY OF BLOOD-BRAIN BARRIER COMPRISING NITRIC OXIDE DONOR AND USE THEREOF**

(30) Priority: 07.02.2020 KR 20200015251
(71) Applicant: POSTECH Research and Business Development Foundation, Pohang-si, Gyeongsangbuk-do 37673 (KR)
(72) Inventor: Kim, Won Jong, 37673 Gyeongsangbuk-do (KR); Kim, Taejeong, 37673 Gyeongsangbuk-do (KR)
(74) Representative: Petraz, Gilberto Luigi

(57) **Abstract**

The present invention relates to a composition to be used for increasing the blood-brain barrier permeability, the composition including a nitric oxide donor, and a use of the composition. More particularly, the invention relates to a composition to be used for increasing the blood-brain barrier permeability, the composition including a nitric oxide donor capable of carrying nitric oxide (NO), wherein the nitric oxide delivered to a site adjacent to the blood-brain barrier by the nitric oxide donor activates matrix metallopeptidase-9 (MMP-9), and the activated MMP-9 weakens a tight junction between a cerebrovascular endothelial cell and a cerebrovascular endothelial cell to increase the blood-brain barrier permeability, and to a use of the composition.

## Description

### BACKGROUND OF THE DISCLOSURE

### Field of the disclosure

The present invention relates to a composition intended to be used to increase the blood-brain barrier (BBB) permeability, the composition including a nitric oxide donor, and a use of the composition. More particularly, the invention relates to a nitric oxide donor-containing composition intended to be used to increase the blood-brain barrier permeability, the composition using a controllable nitric oxide donor so that the delivered nitric oxide temporarily opens the blood-brain barrier to allow a desired substance to be accumulated in the brain, and to a use of the composition.

### Related Art

The blood-brain barrier is a defense system existing in a living body and plays the role of preventing foreign materials such as bacteria and viruses, which have flowed in along the blood vessels, from penetrating into the deep part of the brain. Such a defense system is crucial for protecting the brain; however, there is also a problem that the defense system prevents an artificially administered functional drug from flowing into the brain. There are a method of directly administering a drug into the brain through a surgery and a method of injecting a drug into the cerebrospinal fluid; however, those methods have limitations in terms of high risk, high cost, and low level of convenience for the patient.

Since it was revealed in the late 1980's that nitric oxide (NO) is involved in the blood vessel system, numerous studies on the biological functions of nitric oxide gas molecules have been in active progress.

On the other hand, many drugs have been developed for the treatment of various brain diseases; however, treatments using existing drug systems have significant limitations due to the blood-brain barrier. Therefore, there is a demand for a new composition or drug delivery system, which can deliver a drug in a non-invasive manner but does not interfere with the form or efficacy of the drug.

### SUMMARY

An object of the present invention is to provide a composition or pharmaceutical composition that delivers a nitric oxide donor capable of carrying nitric oxide (NO), which is a blood vessel expanding gas, to the blood-brain barrier, increases the blood-brain barrier permeability, and thereby allows a desired substance to be accumulated in the brain.

In order to solve the problems described above, a composition according to an aspects of the present invention, which is intended to be used to increase the blood-brain barrier permeability, comprises a nitric oxide donor capable of carrying nitric oxide (NO), wherein the nitric oxide that has been delivered to a site adjacent to the blood-brain barrier by the nitric oxide donor activates matrix metallopeptidase-9 (MMP-9), and the activated MMP-9 weakens a tight junction between a cerebrovascular endothelial cell and a cerebrovascular endothelial cell and thereby increases the blood-brain barrier permeability.

At this time, the nitric oxide donor may be any one or more selected from the group consisting of: (a) a NONOate or a derivative thereof, (b) a S-nitrosothiol or a derivative thereof, (c) a metal nitrosyl complex, (d) a BNNs or a derivative thereof, (e) nitrobenzene, (f) an organic nitrate or a derivative thereof and (g) a SIN-1.

The composition may further include an active agent.

Wherein, the active agent may be a small molecule, a protein, a polysaccharide, a nucleic acid, a lipid, or a combination of these.

The nitric oxide donor and the active agent may be administered simultaneously or separately.

The active agent may be a pharmaceutically active agent, a diagnostically active agent, or a combination of these.

Wherein, the composition may be intended to promote the delivery of the active agent into the brain.

Furthermore, the composition may further comprise a pharmaceutically or diagnostically acceptable carrier.

The pharmaceutically active agent may be a drug for treating any one or more diseases selected from the group consisting of a neurodegenerative disease, a neuropsychiatric disease, a brain tumor, a traumatic brain injury, and a stroke, a neurotrophic factor, or a growth factor.

Meanwhile, the pharmaceutical composition according to another aspects of the present invention includes a nitric oxide donor capable of carrying nitric oxide (NO), together with a drug for treating any one or more diseases selected from the group consisting of a neurodegenerative disease, a neuropsychiatric disease, a brain tumor, a traumatic brain injury, and a stroke, a neurotrophic factor, or a growth factor.

At this time, the pharmaceutical composition may further include a diagnostic active agent.

Wherein, the diagnostic active agent may be an imaging agent, a magnetically active agent, or a radioactively active agent.

The pharmaceutical composition may further include a pharmaceutically or diagnostically acceptable carrier.

Furthermore, the nitric oxide donor and the active agent may be administered simultaneously or separately.

According to the present invention of the composition, by regulating the blood-brain barrier permeability with a nitric oxide donor, brain accumulation of a substance that has been simultaneously and non-invasively delivered can be effectively realized.

As a result, the composition according to the present invention can be used as a drug for treating a disease such as a neurodegenerative disease, a neuropsychiatric disease, a brain tumor, a traumatic brain injury, or a stroke, or as a neurotrophic factor or a growth factor.

### BRIEF DESCRIPTION OF DRAWINGS

FIG. 1 is a schematic diagram illustrating the state in which the permeability of the blood-brain barrier is enhanced by nitric oxide, and the state in which as the blood-brain barrier is opened, a substance in the blood vessel is delivered to the brain.
FIG. 2 is a graph showing the results of checking whether a nitric oxide donor according to an embodiment of the present invention has been synthesized, by NMR.
FIG. 3 is a graph showing the results of checking the release amount of nitric oxide in an aqueous solution of a nitric oxide donor according to an embodiment of the present invention.
FIG. 4 is a schematic diagram showing a stage of delivering a nitric oxide donor according to an embodiment of the present invention and Evans blue dye separately by intravenous injection to the blood flow of a mouse and then, after 90 minutes, extracting the brain of the mouse.
FIG. 5 is a photograph showing a mouse brain stained by Evans blue dye that has penetrated into the internal part of the brain.
FIG. 6 shows the data quantitatively indicating the permeability of the blood-brain barrier by using Evans blue dye.

### DESCRIPTION OF EXEMPLARY EMBODIMENTS

Hereinafter, embodiments of the present invention will be described in detail with reference to the attached drawings so that those having ordinary skill in the art to which the present invention is pertained can easily carry out the invention. However, the present invention can be realized in various different forms and is not intended to be limited to the embodiments and drawings described herein.

According to aspects of the present invention, there is provided a composition used for increasing the blood-brain barrier permeability, the composition comprising a nitric oxide donor capable of carrying nitric oxide (NO), wherein the nitric oxide delivered to a site adjacent to the blood-brain barrier by the nitric oxide donor activates MMP-9 (matrix metallopeptidase-9), and the activated MMP-9 weakens a tight junction between a cerebrovascular endothelial cell and a cerebrovascular endothelial cell and increases the blood-brain barrier permeability.

The composition according to the present invention delivers a nitric oxide donor capable of carrying nitric oxide (NO), which is a blood vessel expanding gas, to the blood-brain barrier and increases the blood-brain barrier permeability so that a desired substance can be accumulated in the brain. As a result, the composition can be used as a drug for treating a disease such as a neurodegenerative disease, a neuropsychiatric disease, a brain tumor, a traumatic brain injury, or a stroke, or as a neurotrophic factor or a growth factor.

The nitric oxide donor may be any one or more selected from the group consisting of: (a) a NONOate or a derivative thereof, (b) a S-nitrosothiol or a derivative thereof, (c) a metal nitrosyl complex, (d) a BNNs or a derivative thereof, (e) nitrobenzene, (f) an organic nitrate or a derivative thereof and (g) a SIN-1.

The NONOate can be represented by the following Chemical Formula (1). 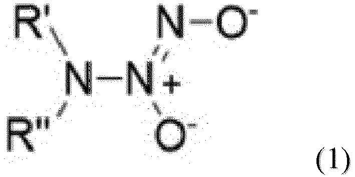 (wherein R' and R" each represent an alkyl group)

Examples of the NONOate include N-diazeniumdiolate, and examples of the derivative of NONOate include DETA-NONOate and pyrrolidine NONOate. The DETA-NONOate and pyrrolidine NONOate can be represented by the following Chemical Formulas (2) and (3), respectively.

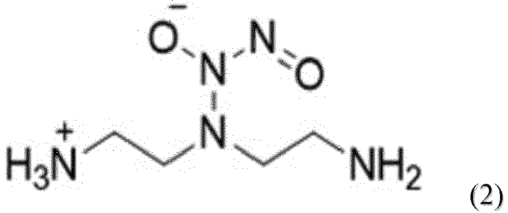

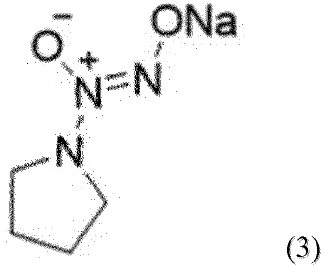

The S-nitrosothiol can be represented by the following Chemical Formula (4), and S-nitroso-L-glutathione, which is a derivative of the S-nitrosothiol, can be represented by the following Chemical Formula (5). 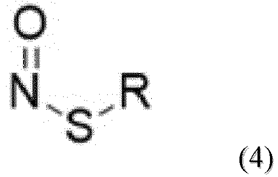 (wherein R represents an organic group)

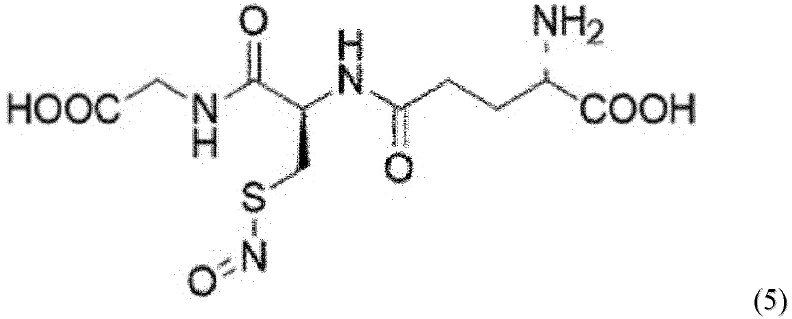

The BNNs can be represented by the following Chemical Formula (6), and BNN6, which is a derivative of the BNNs, can be represented by the following Chemical Formula (7) 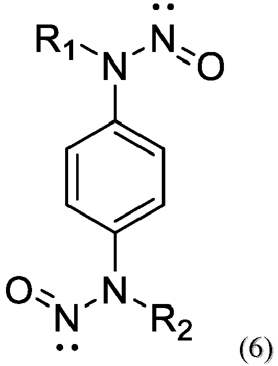 (wherein R' and R" each represent an alkyl group)

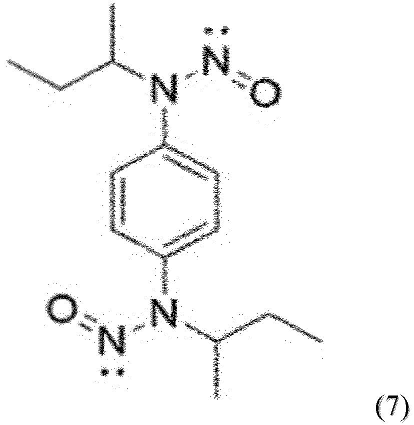

The organic nitrate can be represented by the following Chemical Forumula (8), and nitroglycerin, which is a derivate of the organic nitrate, can be represented by the following Chemical Formula (9) 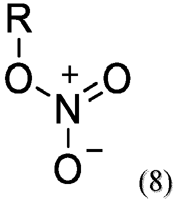 (wherein R represents an organic group)

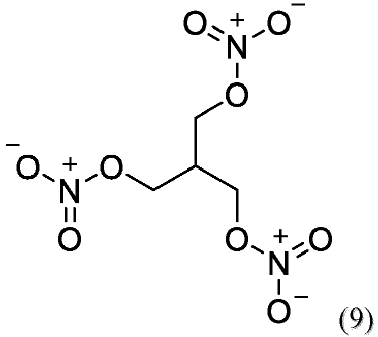

Other nitric oxide donors, namely, a metal nitrosyl complex (wherein the metal is iron (Fe)), a nitrobenzene (metal-substituted) and SIN-1 can be represented by Chemical Formulas (10) to (12), respectively. 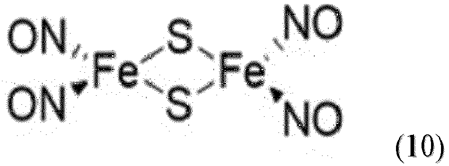 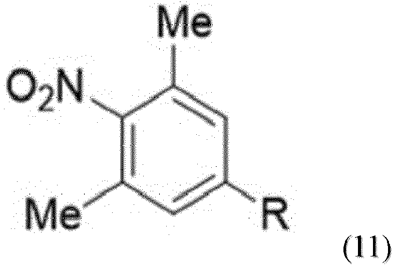 (wherein R represents an organic group)

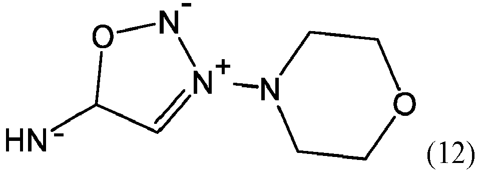

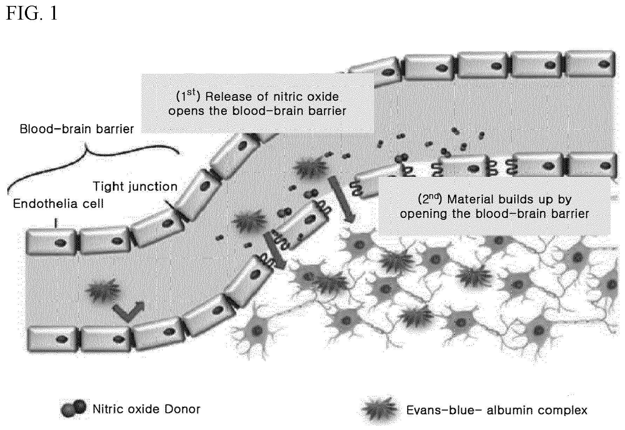

FIG. 1 is a schematic diagram showing the state in which the permeability of the blood-brain barrier is enhanced by nitric oxide, and the state in which the blood-brain barrier is opened, and thereby a substance in the blood vessel is delivered to the brain.

According to FIG. 1, the nitric oxide donor may weaken a tight junction between a cerebrovascular endothelial cell and a cerebrovascular endothelial cell and increase the cerebrovascular permeability. The nitric oxide donor may be used as a permeability enhancer for enhancing the permeability of the inner wall of the brain blood vessels. And the nitric oxide donor may be included in an amount effective for enhancing the permeability of the inner wall of the brain blood vessels. In a case in which the nitric oxide donor is used in combination with an active agent, the nitric oxide door may be administered according to the regimen of administration, for example, the duration of administration, of the active agent.

The brain blood vessels may be blood vessels that have strong tight junctions between vascular endothelial cells and vascular endothelial cells and do not allow or only partially allow substances to pass through the blood vessels. The above-mentioned blood vessels may form the blood-brain barrier (BBB).

The composition may further include an active agent. The active agent may be a substance that is known to be unable to permeate, or is actually unable to permeate, through the inner membrane of the brain blood vessels. Furthermore, the active agent may be a substance that permeates through the inner membrane of the brain blood vessels only in a small amount and cannot be delivered in an effective amount in the absence of other permeation auxiliary agents.

The active agent may be a small molecule, a protein, a polysaccharide, a nucleic acid, a lipid, or a combination of these. The small molecule may be a non-polymer molecule. The molecule may be an organic compound molecule. The protein may be or may not be glycosylated. A glycoprotein may be, for example, erythropoietin (EPO). The protein may be a protein having a molecular weight of 500 Da to 1000 kDa, 500 Da to 500 kDa, 500 Da to 100 kDa, 500 Da to 500 kDa, 1 kDa to 500 kDa, 5 kDa to 500 kDa, 10 kDa to 500 kDa, or 20 kDa to 500 kDa. The polysaccharide may be dextran or starch. The nucleic acid may be a single-stranded or double-stranded polynucleotide. The nucleic acid may be siRNA, shRNA, miRNA, or an antisense oligonucleotide.

The nitric oxide donor and the active agent may be administered simultaneously or separately. The nitric oxide donor and the active agent may be administered as a single composition. The administration may be conducted such that the nitric oxide donor is administered first, followed by administration of the active agent. In this case, the active agent may be administered within 30 minutes, within 20 minutes, within 15 minutes, within 10 minutes, or within 5 minutes, after the administration of the nitric oxide donor. The active agent may be administered, for example, within 1 minute to 30 minutes, within 1 minute to 20 minutes, within 1 minute to 5 minutes, within 5 minutes to 15 minutes, within 5 minutes to 10 minutes, or within 3 minutes to 15 minutes, after the administration of the nitric oxide donor. The administration may be conducted such that the active agent is administered first, followed by administration of the nitric oxide donor. In this case, the nitric oxide donor may be administered within 30 minutes, within 20 minutes, within 15 minutes, within 10 minutes, or within 5 minutes, after the administration of the active agent. The nitric oxide donor may be administered within 1 minute to 30 minutes, within 1 minute to 20 minutes, within 1 minute to 5 minutes, within 5 minutes to 15 minutes, within 5 minutes to 10 minutes, or within 3 minutes to 15 minutes, after the administration of the active agent.

Meanwhile, the active agent may be a pharmaceutically active agent, a diagnostically active agent, or a combination of these.

Wherein, the composition may be intended for promoting the delivery of the active agent into the brain, the spinal cord, or the retina.

The composition may further include a pharmaceutically or diagnostically acceptable carrier. The composition may be a pharmaceutical or diagnostic composition.

Wherein, the pharmaceutically or diagnostically acceptable carrier may be an excipient, a disintegrant, a binding agent, a lubricating agent, or a combination thereof.

The excipient may be microcrystalline cellulose, lactose, low-substituted hydroxycellulose, or a combination thereof. The disintegrant may be sodium starch glycolate, anhydrous dibasic calcium phosphate, or a combination thereof. The binding agent may be polyvinylpyrrolidone, low-substituted hydroxypropyl cellulose, hydroxypropyl cellulose, or a combination thereof. The lubricating agent may be magnesium stearate, silicon dioxide, talc, or a combination thereof.

The pharmaceutically active agent may be a drug for treating any one or more diseases selected from the group consisting of a neurodegenerative disease, a neuropsychiatric disease, a brain tumor, a traumatic brain injury, and a stroke, or a neurotrophic factor or a growth factor.

According to another aspects of the present invention, there is provided a pharmaceutical composition including a nitric oxide donor that can carry nitric oxide (NO), as well as a drug for treating any one or more diseases selected from the group consisting of a neurodegenerative disease, a neuropsychiatric disease, a brain tumor, a traumatic brain injury, and a stroke, a neurotrophic factor, or a growth factor.

At this time, the pharmaceutical composition may further include a diagnostically active agent.

The diagnostically active agent may be an imaging agent, a magnetically active agent, or a radioactively active agent. The imaging agent may be a fluorescent body or a substance producing a fluorescent body. The magnetically active agent may be a magnetic substance such as magnetic particles. The radioactively active agent may be a radioactive atom, molecule, or moiety.

The pharmaceutical composition may further include a pharmaceutically active agent, a diagnostically active agent, or an active agent combining those. The pharmaceutical composition may be a pharmaceutical or a diagnostic composition. The pharmaceutical composition may further include a pharmaceutically or diagnostically acceptable carrier.

The composition or pharmaceutical composition may be formulated into an oral dosage form or a parenteral dosage form. The oral dosage form may be a granular preparation, a powder preparation, a liquid preparation, a tablet, a capsule, a dry syrup, or a combination thereof. The parenteral dosage form ma be an injectable preparation.

According to another aspects of the present invention, there is provided a method for delivering an active agent to an individual, the method comprising a step of administering a nitric oxide donor and the active agent to an individual.

In the above-described method, the nitric oxide donor and the active agent are as described above.

The administration may be carried out by any method known in the pertinent art. The administration may be carried out directly to an individual by any means via a route such as an intravenous, intramuscular, oral, transdermal, transmucosal, intranasal, intratracheal, or subcutaneous administration. The administration may be carried out systemically or topically. The administration may involve applying the drug in a localized manner to the BBB site, for example, to the brain, spinal cord, or retina.

The individual may be a mammal, for example, a human being, a cow, a horse, a pig, a dog, a sheep, a goat, or a cat. The individual may be an individual having a disease in the brain, spinal cord, or retina.

The administration may be carried out by administering a nitric oxide donor and an active agent respectively in an amount of 0.01 mg to 1,000 mg, for example, 0.1 mg to 500 mg, 0.1 mg to 100 mg, 0.1 mg to 50 mg, 0.1 mg to 25 mg, 1 mg to 1,000 mg, 1 mg to 500 mg, 1 mg to 100 mg, 1 mg to 50 mg, 1 mg to 25 mg, 5 mg to 1,000 mg, 5 mg to 500 mg, 5 mg to 100 mg, 5 mg to 50 mg, 5 mg to 25 mg, 10 mg to 1,000 mg, 10 mg to 500 mg, 10 mg to 100 mg, 10 mg to 50 mg, or 10 mg to 25 mg, per day for each individual.

The nitric oxide donor and the active agent may be administered simultaneously or separately. The nitric oxide donor and the active agent may be administered as a single composition or as separate compositions. The administration may be carried out such that the nitric oxide donor is administered first, followed by administration of the active agent. In this case, the active agent may be administered within 30 minutes, within 20 minutes, within 15 minutes, within 10 minutes, or within 5 minutes, after the administration of the nitric oxide donor. The active agent may be administered, for example, within 1 minute to 30 minutes, within 1 minute to 20 minutes, within 1 minute to 5 minutes, within 5 minutes to 15 minutes, within 5 minutes to 10 minutes, or within 3 minutes to 15 minutes, after the administration of the nitric oxide donor. The administration may be conducted such that the active agent is administered first, followed by administration of the nitric oxide donor. In this case, the nitric oxide donor may be administered within 30 minutes, within 20 minutes, within 15 minutes, within 10 minutes, or within 5 minutes, after the administration of the active agent. The nitric oxide donor may be administered, for example, within 1 minute to 30 minutes, within 1 minute to 20 minutes, within 1 minute to 5 minutes, within 5 minutes to 15 minutes, within 5 minutes to 10 minutes, or within 3 minutes to 15 minutes, after the administration of the active agent.

The above-described method may be intended to diagnose the presence of a substance or a symptom, or to treat a symptom. The substance or symptom may be present in the brain, the retina, or the spinal cord.

The active agent may be a pharmaceutically active agent or a diagnostically active agent. The method may include administering a pharmaceutically or diagnostically acceptable carrier together.

The nitric oxide donor may be administered in an amount effective for enhancing the permeability of the blood-brain barrier.

According to another aspects of the present invention, there is provided a use of a nitric oxide donor for being used in a method for delivering an active agent to an individual. This use may be intended to deliver an active agent to an individual through the brain blood vessels.

Hereinafter, the present invention will be described in more detail by way of specific Examples. The following Examples are only for the purpose of illustrating the present invention, and the present invention is not intended to be limited by the following Examples.

### Manufacturing Example: Synthesis of nitric oxide donor

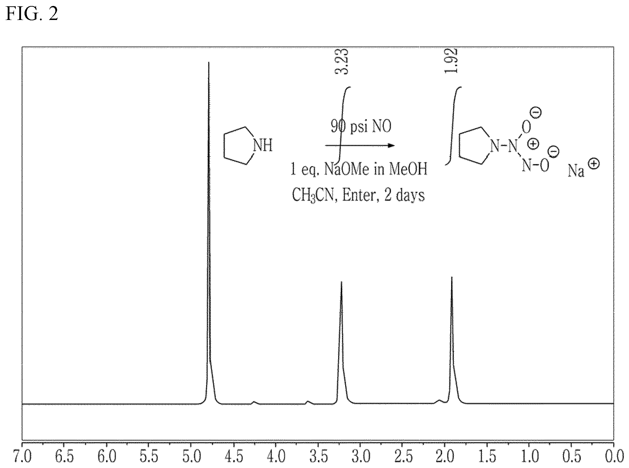

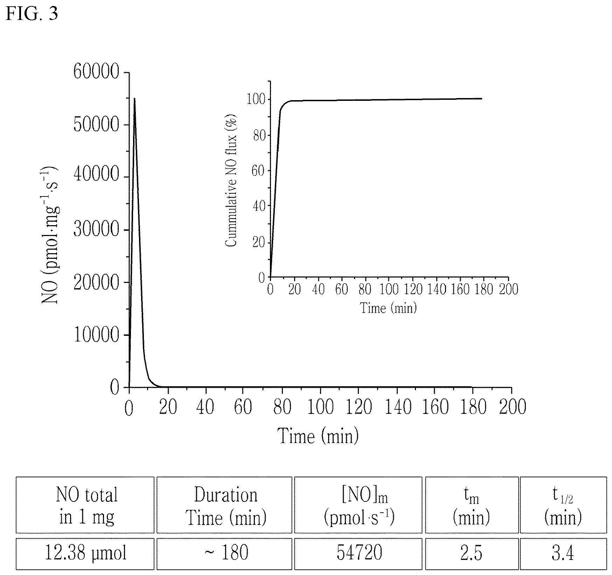

In order to introduce nitric oxide to a secondary amine group of pyrrolidine, nitric oxide gas at 90 psi was caused to react with pyrrolidine for two days in a solvent mixture of methanol, acetonitrile, and diethyl ether including 1 equivalent of sodium methoxide, and thus pyrrolidine NONOate represented by Chemical Formula (3) was synthesized. Whether the nitric oxide donor was synthesized was checked by NMR (see FIG. 2) and was also checked by determining the release amount of nitric oxide in an aqueous solution by a chemiluminescence method (see FIG. 3).

### Experimental Example: Verification of permeation through blood-brain barrier using Evans blue

Evans blue is one of the dyes exhibiting blue color and is mainly used for checking the permeability of the blood-brain barrier. Evans blue strongly binds to albumin, and a macromolecule thus formed by binding cannot permeate through the blood-brain barrier.

Therefore, the permeability of the blood-brain barrier can be checked in a small animal by checking the presence or absence of brain accumulation of Evans blue resulting from the delivery of nitric oxide. Through this, the functionality of the blood-brain barrier permeation system using nitric oxide can be quantitatively checked.

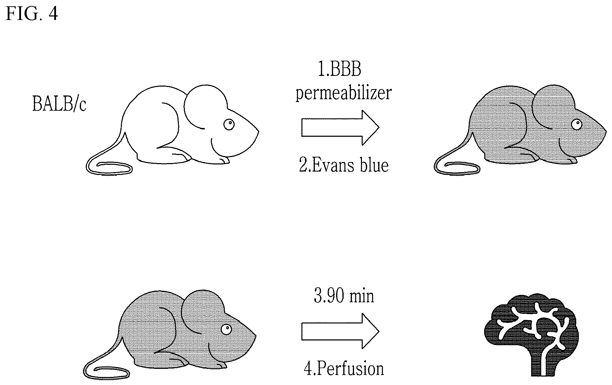

FIG. 4 is a schematic diagram showing a step of separately delivering a nitric oxide donor and Evans blue dye to the blood flow of a mouse through intravenous injection and then extracting the brain of the mouse after 90 minutes.

More particularly, 100 µL of a nitric oxide donor and 100 µL of 2 wt% Evans blue dye were delivered to the blood flow of each of eight-week old Balb/c mice separately through intravenous injection (IV). After 90 minutes, blood that could obstruct measurement was removed by perfusion, and the brain was extracted.

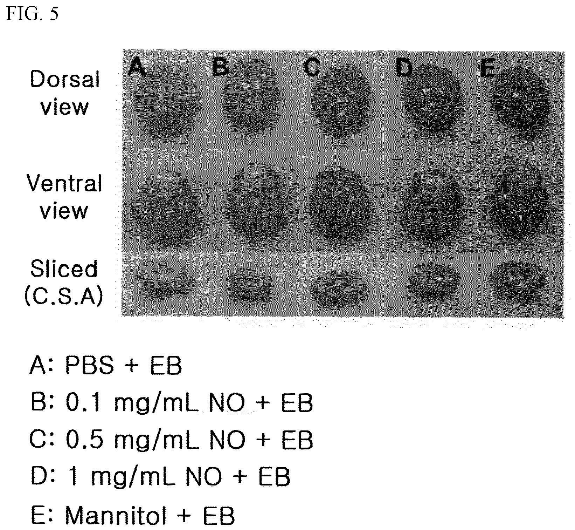

FIG. 5 is a photograph showing a mouse brain stained with Evans blue dye that had permeated into the internal part of the brain.

To explain this with reference to FIG. 5, whether Evans blue actually permeates through the blood-brain barrier by a nitric oxide donor synthesized as described above was checked by using PBS (A) as a negative control group, using mannitol (E) as a positive control group, and varying the concentration of the nitric oxide donor (B, C, and D). Evans blue that had been delivered together with a nitric oxide donor promoting the blood-brain barrier permeation, permeated through the blood-brain barrier and into the deep part of the brain, and this could be verified by staining of the extracted brain after perfusion.

In addition, Evans blue that had been delivered together with the nitric oxide donor and stained the brain was quantitatively measured, and the blood-brain barrier permeability was determined.

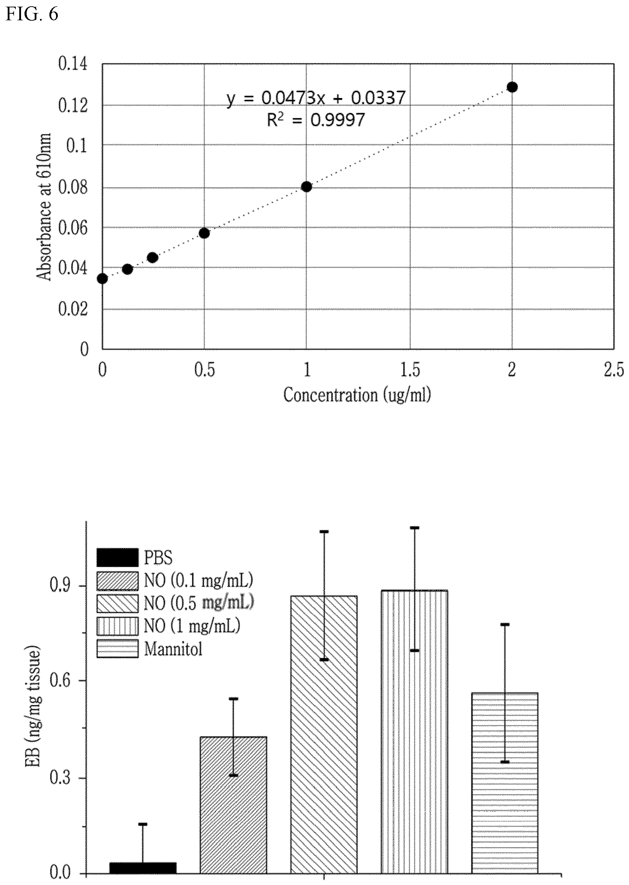

FIG. 6 shows the data quantitatively indicating the permeability of the blood-brain barrier by using Evans blue dye. To explain this with reference to FIG. 6, the amount of Evans blue accumulated in the brain was quantitatively determined by a UV-Vis absorbance quantification method using the absorbance at a wavelength of 610 nm. It was verified that the nitric oxide donor disintegrated the blood-brain barrier much more efficiently than 100 mg/mL of mannitol, which was the positive control group.

As a result of the experiment described above, Evans blue dye that had entered the brain through the blood-brain barrier opening action by means of the nitric oxide donor could be recognized. Such action of nitric oxide is expected to be applicable to the treatment of various brain diseases by effectively delivering to the brain various low-molecular weight drugs, proteins, and therapeutic substances, which could not be delivered due to the blood-brain barrier. The nitric oxide donor can be further developed into various forms.

The above-given description is intended only for illustrating the present invention, and any person having ordinary skill in the art to which the present invention is pertained, will be able to understand that the present invention can be realized in any form that has been modified to the extent that the fundamental characteristics of the present invention are maintained. Therefore, the disclosed Examples and Experimental Examples should be considered not from a restrictive viewpoint but from an illustrative viewpoint. The scope of the present invention is disclosed not in the above-described embodiments but in the following claims, and it should be understood that any alterations made to an extent equivalent to the claims are included in the present invention.

## Claims

1. A composition for use to increase the blood-brain barrier permeability, comprising a nitric oxide donor capable of carrying nitric oxide (NO),
wherein nitric oxide delivered to a site adjacent to the blood-brain barrier by the nitric oxide donor activates matrix metallopeptidase-9 (MMP-9), and
the activated MMP-9 weakens a tight junction between a cerebrovascular endothelial cell and a cerebrovascular endothelial cell to increase the blood-brain barrier permeability.

2. The composition according to claim 1, the nitric oxide donor is any one or more selected from the group consisting of: (a) a NONOate or a derivative thereof, (b) a S-nitrosothiol or a derivative thereof, (c) a metal nitrosyl complex, (d) a BNNs or a derivative thereof, (e) nitrobenzene, (f) an organic nitrate or a derivative thereof and (g) a SIN-1.

3. The composition according to claim 1, the composition further includes an active agent.

4. The composition according to claim 3, the active agent is a small molecule, a protein, a polysaccharide, a nucleic acid, a lipid, or a combination of thereof.

5. The composition according to claim 3, the nitric oxide donor and the active agent are administered simultaneously or separately.

6. The composition according to claim 3, the active agent is a pharmaceutically active agent, a diagnostically active agent, or a combination of thereof.

7. The composition according to claim 6, the composition is intended for promoting the delivery of the active agent into the brain.

8. The composition according to claim 6, the composition further includes a pharmaceutically or diagnostically acceptable carrier.

9. The composition according to claim 6, the pharmaceutically active agent is a drug for treating any one or more diseases selected from the group consisting of a neurodegenerative disease, a neuropsychiatric disease, a brain tumor, a traumatic brain injury, and a stroke, a neurotrophic factor, or a growth factor.

10. A pharmaceutical composition, comprising:
a nitric oxide donor capable of carrying nitric oxide (NO); and
a drug for treating any one or more diseases selected from the group consisting of a neurodegenerative disease, a neuropsychiatric disease, a brain tumor, a traumatic brain injury, and a stroke, a neurotrophic factor, or a growth factor.

11. The pharmaceutical composition according to claim 10, the pharmaceutical composition further includes a diagnostically active agent.

12. The pharmaceutical composition according to claim 11, the diagnostically active agent is an imaging agent, a magnetically active agent, or a radioactively active agent.

13. The pharmaceutical composition according to claim 10, the pharmaceutical composition further includes a pharmaceutically or diagnostically acceptable carrier.

14. The pharmaceutical composition according to claim 11, the nitric oxide donor and the active agent are administered simultaneously or separately.
